# FASCICULE DE BREVET EUROPEEN

(11) **EP 2 725 993 B1**
(45) Date de publication et mention de la délivrance du brevet: **04.03.2015**
(21) Numéro de dépôt: 12738485.7
(22) Date de dépôt: 27.06.2012
(51) Int. Cl.: A61B 17/70, A61B 17/84

(54) **DISPOSITIF DE FIXATION VERTÉBRALE**
VORRICHTUNG FÜR WIRBELFIXIERUNG
VERTEBRAL FIXATION DEVICE

(30) Priorité: 30.06.2011 FR 1102072
(43) Date de publication de la demande: 07.05.2014
(73) Titulaire: Implanet, Société Anonyme, 33650 Martillac (FR)
(72) Inventeur: LE COUËDIC, Régis, F-33000 Bordeaux (FR); BACCELLI, Christian, F-33650 Saucats (FR)
(74) Mandataire: Benech, Frédéric
(86) Numéro de dépôt international: PCT/FR2012/000259
(87) Numéro de publication internationale: WO 2013/001180

(56) Documents cités:
- WO-A1-2008/146185
- FR-A1- 2 662 073
- FR-A1- 2 890 850
- US-A- 6 086 590
- US-B1- 6 554 831

## Description

La présente invention concerne un dispositif de fixation vertébrale pour maintien d'une vertèbre rachidienne sur une tige, comportant un corps de section transversale en forme de U ou sensiblement en forme de U, une bande souple de liaison de ladite vertèbre avec le corps de fixation muni de fentes de passage de ladite bande et des moyens de blocage réglable de la bande souple contre la tige dans ledit corps de fixation.

Elle trouve une application particulièrement importante bien que non exclusive dans le domaine du redressement de la colonne vertébrale d'un patient présentant une courbure anormale.

Dans ce cas, les vertèbres n'étant pas alignées correctement les unes par rapport aux autres par rapport à l'axe vertébral, elles présentent des inclinaisons entre elles.

A certains endroits, les bords latéraux des vertèbres vont donc être d'un côté rapprochés les uns des autres, et de l'autre côté éloignés les uns des autres.

Afin de redresser l'ensemble, il est connu de remettre à une distance sensiblement équivalente les bords latéraux des vertèbres de part et d'autre de la colonne vertébrale, par le biais de tiges reliant entre elles, soit des vis, que l'on insère dans les vertèbres elles-mêmes, soit des crochets, que l'on introduit le long du canal rachidien.

De tels dispositifs présentent cependant des inconvénients.

L'utilisation de vis n'est tout d'abord possible que si les vertèbres sont en bon état et/ou suffisamment larges au niveau de la fixation.

L'utilisation de crochets est quant à elle très délicate puisque l'opérateur ne doit pas toucher la moelle épinière sous peine de paralysie du patient.

Pour pallier à ces inconvénients il a été proposé (FR 02 09 317 publié sous le n° FR 2 842 724 ou FR 06 50 609 publié sous le n° FR 2 890 850) un système permettant d'éviter les vis de fixation ou les crochets.

Le système comprend un lien flexible de fixation de la vertèbre sur une pièce de liaison elle-même fixée à la tige de redressement.

Des moyens de blocage du lien flexible par refermeture de la pièce de liaison sur la tige sont prévus. Ce système présente cependant ici encore des inconvénients.

Il nécessite en effet une articulation de la pièce de liaison de façon à permettre l'insertion latérale de la tige et du lien souple. Le lien est par ailleurs susceptible de glisser sur la vertèbre et/ou de ne plus l'enserrer du fait de la mise en tension d'autres pièces de liaison avec d'autres vertèbres situées à proximité et/ou le long de la tige.

On connaît également (US 6,086,590) un dispositif de connexion de câble sur une tige chirurgicale permettant de garantir une bonne fixation, le câble étant introduit de façon connue autour de l'os avant d'être resserré.

Le document US 6,554,831 décrit quant-à-lui un système de fixation sur la tige par compression d'une pièce en U lors du vissage dans l'os.

Il est également connu (WO 2008/146185) un système du type serre joint à crans classiquement utilisé dans le domaine électrique.

Tous ces dispositifs présentent des inconvénients notamment parce qu'ils sont lourds et/ou difficiles à mettre en oeuvre risquant de ce fait de blesser le patient.

La présente invention décrit un dispositif de fixation vertèbrale pour maintien d'une vertèbre rachidienne sur une tige selon la revendication indépendante 1. Des réalisations préférées de l'invention sont décrites dans les revendications dépendantes.

La présente invention vise à fournir un dispositif de fixation vertébrale répondant mieux que ceux antérieurement connus aux exigences de la pratique, notamment en ce qu'elle va permettre une plus grande flexibilité, une meilleure solidité du fait d'une absence d'articulation mécanique toujours susceptible de blocage, en ce qu'elle présente des possibilités de réglage très améliorées, et une absence de risque de glissement et/ou de perte de tension sur les vertèbres, et ce pour un coût moindre.

Elle présente également l'avantage d'un maniement aisé de la bande ou lien souple, permettant son insertion facile par le chirurgien autour de la vertèbre, ce qui va autoriser par exemple une fixation efficace, notamment par le biais d'un noeud en tête d'alouette, impossible ou très difficile à pratiquer avec le dispositif de l'art antérieur.

Dans ce but, l'invention propose essentiellement un dispositif de fixation vertébrale pour maintien d'une vertèbre rachidienne sur une tige, comportant ladite tige, un corps de fixation, de section transversale en forme de U ou sensiblement en forme de U, une bande souple de liaison de ladite vertèbre avec le corps de fixation, ledit corps de fixation comportant deux fentes de passage de ladite bande et des moyens de blocage réglable de la bande souple contre la tige dans ledit corps de fixation, caractérisé en ce que la bande souple forme une boucle fermée sur elle-même munie d'une lamelle souple, ladite lamelle étant propre à former un guide de passage autour de la vertèbre et dans le corps de fixation.

Avantageusement la lamelle souple est déformable par exemple pour adopter une forme en crochet permettant une insertion plus facile et précise.

De cette façon le chirurgien peut la conformer de façon modifiable manuellement pour mieux passer autour de la vertèbre sans risquer de blesser le patient, la lamelle étant par exemple en métal souple, relativement mou conservant sa forme par flexion.

Ainsi et avec l'invention, grâce notamment à la grande facilité d'enfilage et/ou de mise en place de la bande, une excellente fixation sur la vertèbre est possible. L'existence de la boucle permet par ailleurs le passage de la lamelle au travers d'elle-même pour bloquer la bande sur la vertèbre.

Avantageusement, pour former la boucle fermée, la bande présente une première extrémité fixée directement, par exemple par couture ou collage sur l'autre extrémité, ou sur une portion d'extrémité elle-même terminée par ladite autre ou seconde extrémité.

En d'autres termes, pour former la boucle, une extrémité de celle-ci est fixée de façon inamovible sur une autre partie (extrémité ou point intermédiaire) de ladite bande par collage ou couture.

Dans des modes de réalisation avantageux, on a de plus recours à l'une et/ou à l'autre des dispositions suivantes :
- La bande souple comprend un noeud en tête d'alouette, de fixation sur la vertèbre, les deux brins issus du noeud passant d'un même côté de la tige dans le corps de fixation, et étant propres à être sectionnés à leur sortie sensiblement au ras du corps en amont de la lamelle souple ;
- la lamelle est incluse dans la boucle ;
- la lamelle est fixée à l'extérieur de la boucle ;
- les moyens de blocage réglable sont formés par une pièce de liaison reliant les extrémités en vis à vis des deux branches du U, et les fentes du corps de fixation, de passage de ladite bande sont formées respectivement chacune par un évidement en vis à vis situé du côté du fond du U, les moyens de blocage étant agencés pour comprimer la tige contre la paroi et les évidements en vis à vis étant situés du côté du fond du U pour le passage entre ledit fond et la tige ;
- le corps est formé d'une seule pièce ;
- le fond du U formant une paroi de fond du U, celle-ci est en forme de demi cylindre de forme complémentaire à celle de la tige, terminé de part et d'autre par des rebords longitudinaux autorisant un clipsage de la tige dans le fond du U grâce à une déformation des branches ;
- la pièce de liaison est formée par une vis de serrage munie d'un côté d'une tête de passage dans une première branche du U, ladite tête comportant une partie tronconique agencée pour comprimer la tige au fur et à mesure de son vissage et de l'autre côté d'une extrémité de vissage sur la branche du U opposée ;
- Le corps de section transversale en forme de U présentant une première branche du U comprenant une face externe, la tête de la vis de serrage comporte de plus un épaulement propre à coopérer avec la face externe de la première branche du U ;
- le corps de fixation est en matériau polymère, la tige et la pièce de liaison étant en titane et la bande souple étant une tresse en polymère.

Un procédé utilisant un dispositif tel que décrit ci-dessus est décrit ci dessous. Ce procédé n'est pas revendiqué.

Elle concerne aussi un procédé de fixation d'une vertèbre rachidienne sur une tige, à l'aide d'un dispositif comportant un corps de section transversale en forme de U ou sensiblement en forme de U, une bande souple de liaison de ladite vertèbre avec le corps de fixation muni de fentes de passage de ladite bande et des moyens de blocage réglable de la bande souple contre la tige dans ledit corps de fixation,
caractérisé en ce que la bande souple comprenant une boucle fermée sur elle-même munie d'une lamelle souple à une extrémité,
on passe une extrémité de la boucle pliée sur elle-même autour d'une branche de la vertèbre du côté opposé à la lamelle, pour former une virole à l'extérieur de la vertèbre et on fait passer la lamelle souple au travers de la virole pour former un noeud en tête d'alouette autour de ladite branche, que l'on serre,
on introduit ladite lamelle dans les fentes de passage du corps de fixation démuni de tige,
on introduit la tige latéralement dans le corps pour comprimer les deux brins de la boucle contre le fond du corps, que l'on maintien à léger frottement dans le corps,
on fait glisser le corps en position à proximité de la vertèbre,
on met en tension la bande souple pour ramener la vertèbre près de la tige,
puis on comprime la tige contre la paroi par les moyens de blocage, pour fixation définitive, avant de sectionner les brins de la boucle dépassant dudit corps.

Avantageusement on visse une vis de serrage munie d'un côté d'une tête de passage dans une première branche du U, ladite tête comportant une partie tronconique agencée pour comprimer la tige au fur et à mesure de son vissage et de l'autre côté d'une extrémité de vissage sur la branche du U opposée.

L'invention sera mieux comprise à la lecture de la description qui suit de modes de réalisation donnés ci-après à titre d'exemples non limitatifs. La description se réfère aux dessins qui l'accompagnent dans lesquels :
- La figure 1 est une vue en perspective d'un principe de fixation de la bande souple selon l'invention.
- La figure. 2 montre un mode de réalisation du dispositif sur une vertèbre selon l'invention.
- Les figures 3 et 4 sont des vues en perspectives d'une bande selon deux modes de réalisation de l'invention.
- La figure 5 est une vue en coupe du corps, des moyens de blocage et d'une tige utilisée avec un dispositif selon le mode de réalisation de l'invention plus particulièrement décrit.
- La figure 6 montre, en vue de dessus, le dispositif de la figure 1 d'un côté en train d'être fixé, et de l'autre côté fixé, sur une vertèbre.

Dans la suite de la description on utilisera les mêmes numéros de références pour désigner les mêmes éléments.

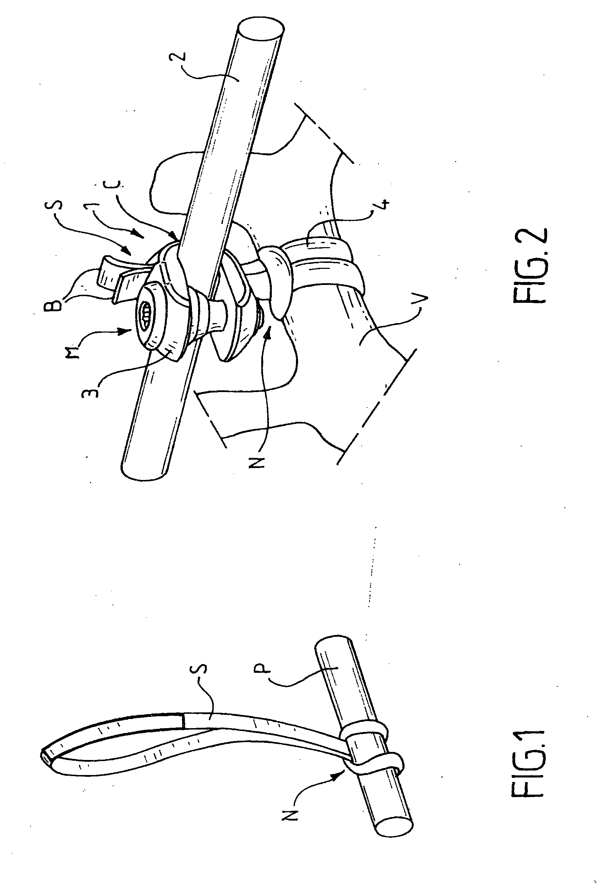

La figure 1 donne le principe du noeud N à tête d'alouette tel que réalisé avec une bande ou sangle S autour d'une pièce tubulaire P, qui autorise une fixation ferme et bloquée de l'une sur l'autre.

La figure 2 montre un dispositif 1 de fixation vertébrale pour maintien d'une vertèbre V rachidienne (partiellement représentée) sur une tige cylindrique 2.

Le dispositif comporte un corps 3 de fixation sur la tige et une bande souple 4 en polymère tressé, par exemple en polyester de 6 mm de large et de trente centimètres de long.

La bande souple 4 forme par ailleurs un noeud à tête d'alouette N, de fixation sur la vertèbre V, les deux brins B du noeud passant d'un même côté C de la tige 2 dans le corps 3 de fixation, et étant sectionné à leur sortie S sensiblement au ras du corps.

Il comprend également des moyens M de blocage réglable de la bande souple sur le corps de fixation 3.

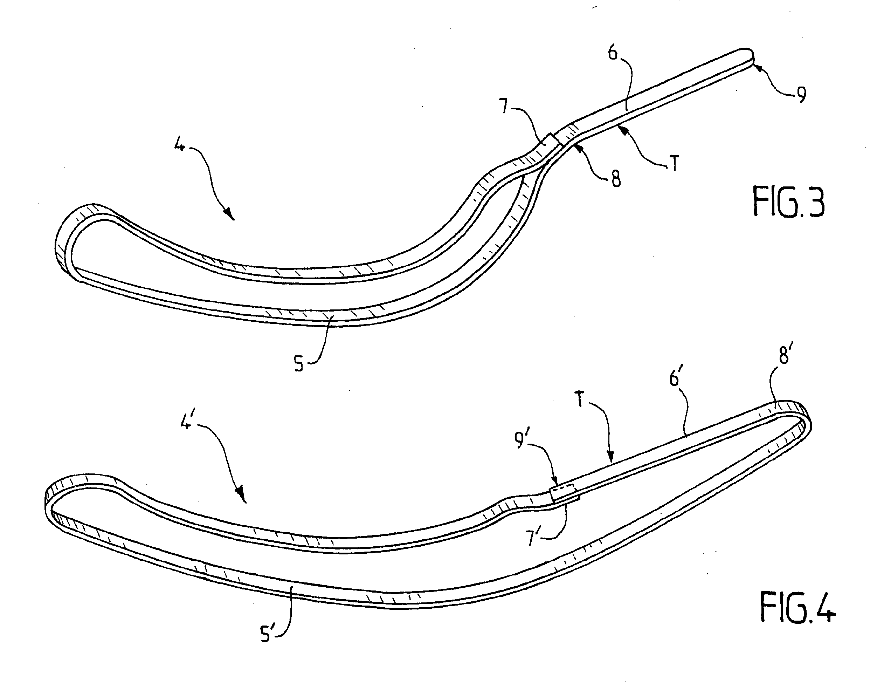

En référence aux figures 3 et 4, la bande souple 4, 4' comprend une boucle fermée 5, 5' munie d'une lamelle souple 6, 6' soit à l'extérieur de la boucle (figure 3) soit incluse dans la boucle (figure 4).

La lamelle est par exemple en acier souple très fin, de 0,1 mm à 0,5 mm d'épaisseur, par exemple insérée dans la tresse T de la bande souple, l'extrémité 7, 7' de la bande sans lamelle étant reliée à la lamelle par exemple en étant fixée par collage ou cousue d'un côté 8 dit interne de la lamelle (figure 3) ou de l'autre côté 9' dit externe de la lamelle (figure 4).

Sur la figure 2 on voit que la bande souple 4 comprend un noeud d'alouette N, de fixation sur la vertèbre.

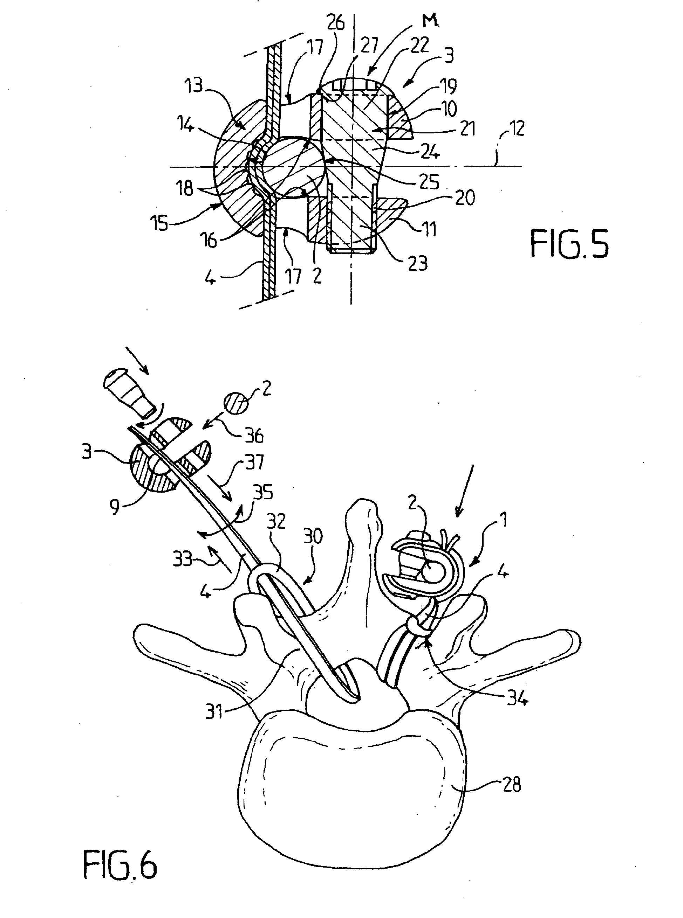

Plus précisément en référence à la figure 5, le corps 3 est formé par une pièce d'un seul tenant formant une pince présentant une section transversale en forme de U, ledit U comprenant deux branches épaisses 10 et 11 de section transversale sensiblement en forme de demi ovale, symétriques par rapport à un plan longitudinal 12, et reliées entre elles par une partie de liaison 13 en forme de demi anneau torique formant d'un côté le fond 14 en demi cylindre du U, et de l'autre côté une paroi externe 15 arrondie de surface torique.

La paroi de fond du U est de forme complémentaire à celle de la tige 2 ou sensiblement complémentaire, et comprend des lèvres ou rebords longitudinaux 16 autorisant un clipsage de la tige dans le fond du U une fois la tresse 4 passée en double épaisseur (voir aussi figure 2).

Chaque branche 10, 11 comporte un évidement 17, par exemple en forme de fente large, par exemple 5 à 10 fois plus large que l'épaisseur de la tresse pour faciliter son introduction lors de l'opération.

Le fond 14 du U comporte de plus des crans 18 de blocage, anti-retour, parallèles au plan longitudinal 12 et présentant de façon connue en elle-même des arêtes formant des angles opposés ou perpendiculaires au sens de glissement, s'opposant au desserrement de la boucle une fois que le serrement est exercé.

Chaque branche 10 et 11 comporte un orifice cylindrique respectivement 19 et 20 de passage des moyens M de blocage, à savoir un alésage 19 de diamètre D et un orifice cylindrique taraudé 20 de vissage de diamètre d < D.

Les moyens M de blocage sont formés par une pièce de liaison 21, ou vis, munie d'un côté d'une tête 22 de passage dans l'alésage 19 du U, et de l'autre côté d'une extrémité 23 de vissage dans l'orifice taraudé 20.

La tête 22 de la pièce 21 comprend une partie supérieure cylindrique qui coopère à frottement doux avec l'alésage 19, ladite partie supérieure étant connectée de façon solidaire avec une partie inférieure tronconique 24, se rétrécissant vers le bas et agencée pour comprimer (appui 25 sur la figure 5) la tige 2 au fur et à mesure du vissage de la pièce.

Dans le mode de réalisation plus particulièrement décrit ici, la tête de la vis comporte de plus un épaulement 26, par exemple tronconique, propre à coopérer avec la face externe 27 de la première branche du U et à servir de butée d'arrêt du vissage.

On va maintenant décrire en référence à la figure 6 la mise en place du dispositif 1 sur la vertèbre 28.

Le dispositif 1 décrit ci-avant permet de connecter mécaniquement la tresse souple 4 sur la tige d'union métallique 2 avec la vertèbre 28.

Cet implant est particulièrement indiqué dans le cadre d'une chirurgie du rachis de type scoliose.

On passe tout d'abord (partie gauche de la figure) l'extrémité 30 de la boucle pliée sur elle-même autour d'une branche 31 de la vertèbre 28, pour former une virole 32 à l'extérieur de la vertèbre par rapport à la branche 31 (ici la lame transverse, arc postérieur).

On fait alors passer la lamelle 6 au travers de la virole comme une aiguille au travers de la boucle que l'on serre (flèche 33) pour former le noeud en tête d'alouette 34 (voir côté droit de la figure).

Puis la tresse 4 est introduite dans le corps 3 au travers des fentes 17.

Après rotation (flèche 35) de la bande, on introduit alors la tige 2 (flèche 36) latéralement dans le corps 3 pour comprimer les deux brins B de la boucle contre le fond 14 avec encliquage, ce qui d'une part laisse une possibilité de glissement compte tenu de la relative élasticité du corps, et d'autre part fixe le corps sur la tige ce qui lui évite de tomber.

On fait alors glisser le corps flèche 37 en position à proximité de la vertèbre (voir partie droite). Puis on met en tension la bande en tirant sur celle-ci au travers du corps, avec un ancillaire adéquat, et enfin on comprime la tige sur la paroi du fond par vissage.

La même opération est réalisée sur la ou les vertèbres d'à côté que l'on souhaite repositionner les unes par rapport aux autres, pour ici réparer la scoliose.

On va décrire uniquement ci-après la fixation d'un seul corps, les fixations des autres étant effectuées de la même façon une fois leur position respective fixée le long de la tige.

La vis 22 est insérée dans l'alésage 16 puis commence à être vissée dans l'alésage 19.

Le vissage est effectué par le chirurgien qui a accès aux têtes de vis chacune munie d'un orifice et/ou évidement de vissage 28 connu en lui-même.

Une fois l'ensemble prépositionné et alors qu'il reste du jeu sur les tresses, celles-ci sont tirées de façon à venir serrer les boucles sur les lames des vertèbres respectives.

Les arêtes anti-retour 18 permettent le serrage dans un sens et empêchent le desserrage dans l'autre lorsque les deux extrémités de la bande souple restent encore un peu mobiles.

Puis le vissage des vis 22 permet aux parties coniques 24 de venir progressivement en appui sur la tige d'union 2 jusqu'à verrouiller l'ensemble en bloquant pour chaque dispositif par serrage la tresse 4 entre le fond du U et la tige d'union.

Comme il va de soi et comme il résulte également de ce qui précède, la présente invention n'est pas limitée aux modes de réalisation plus particulièrement décrits. Elle en embrasse au contraire toutes les variantes et notamment celles où deux ou plusieurs tiges sont fixées à la suite ou de part et d'autre de la colonne vertébrale.

## Revendications

1. Dispositif (1) de fixation vertébrale pour maintien d'une vertèbre (V) rachidienne sur une tige (2), comportant ladite tige, un corps (3) de fixation, de section transversale en forme de U ou sensiblement en forme de U, une bande souple (4, 4') de liaison de ladite vertèbre avec le corps de fixation, ledit corps de fixation comportant deux fentes (17) de passage de ladite bande et des moyens (M) de blocage réglable de la bande souple contre la tige dans ledit corps de fixation,
**caractérisé en ce que** la bande souple (4, 4') forme une boucle (5, 5') fermée sur elle-même munie d'une lamelle (6, 6') déformable en métal souple conservant sa forme par flexion, de sorte qu'elle est propre à former un guide de passage autour de la vertèbre et dans le corps de fixation.

2. Dispositif selon la revendication 1, **caractérisé en ce que** la bande souple (4, 4') comprend un noeud (N) en tête d'alouette, de fixation sur la vertèbre, les deux brins (B) issus du noeud passant d'un même côté de la tige (2) dans le corps (3) de fixation via les fentes (17) de passage.

3. Dispositif selon l'une quelconque des revendications précédentes, **caractérisé en ce que** la lamelle (6') est incluse dans la boucle (5').

4. Dispositif selon l'une quelconque des revendications 1 et 2, **caractérisé en ce que** la lamelle (6) est fixée à l'extérieur de la boucle (5).

5. Dispositif selon l'une quelconque des revendications précédentes, **caractérisé en ce que** les moyens (M) de blocage réglable sont formés par une pièce de liaison (21) reliant les extrémités en vis à vis des deux branches du U,
**en ce que** les fentes (17) du corps de fixation, de passage de ladite bande, sont formées respectivement chacune par un évidement en vis à vis situé du côté du fond du U, les moyens (M) de blocage étant agencés pour comprimer la tige contre la paroi du fond du U et les évidements en vis à vis étant situés du côté du fond du U pour le passage entre ledit fond et la tige.

6. Dispositif selon la revendication 5, **caractérisé en ce que** le corps (3) est formé d'une seule pièce.

7. Dispositif selon la revendication 6, **caractérisé en ce que** le fond du U formant une paroi de fond (14), ladite paroi est en forme de demi cylindre de forme complémentaire à celle de la tige, terminé de part et d'autre par des rebords longitudinaux (16) autorisant un clipsage de la tige (2) dans le fond du U grâce à une déformation des branches.

8. Dispositif selon l'une quelconque des revendications 5 à 7, **caractérisé en ce que** la pièce de liaison (21) est formée par une vis de serrage munie d'un côté d'une tête (22) de passage dans une première branche du U, ladite tête comportant une partie tronconique (24) agencée pour comprimer la tige au fur et à mesure de son vissage et de l'autre côté d'une extrémité (23) de vissage sur la branche du U opposée.

9. Dispositif selon la revendication 8, **caractérisé en ce que** le corps (3) de section transversale en forme de U présente une première branche du U comprenant une face externe et, **en ce que** la tête (22) de la vis de serrage comporte de plus un épaulement (26) propre à coopérer avec la face externe (27) de la première branche du U.

10. Dispositif selon l'une quelconque des revendications précédentes, **caractérisé en ce que** le corps (3) de fixation est en matériau polymère, la tige (2) et la pièce de liaison (21) étant en titane et la bande souple (4) étant une tresse en polymère.

## Patentansprüche

1. Wirbelsäulenfixierungsvorrichtung (1) zum Halten eines Wirbelsäulenwirbels (V) an einem Stift (2), umfassend den Stift, einen Fixierungskörper (3) mit einem U-förmigen oder im Wesentlichen U-förmigen Querschnitt, ein flexibles Band (4, 4') für die Verbindung des Wirbelknochens mit dem Fixierungskörper, wobei der Fixierungskörper zwei Schlitze (17) für die Durchführung des Bandes und Mittel (M) für die einstellbare Arretierung des flexiblen Bandes an dem Stift in dem Fixierungskörper aufweist,
**dadurch gekennzeichnet,**
**dass** das flexible Band (4, 4') eine in sich geschlossene Schlaufe (5, 5') bildet, die mit einer verformbaren Lamelle (6, 6') aus flexiblem Metall versehen ist, welche ihre Form durch Biegung behält, so dass sie geeignet ist, eine Durchlaufführung um den Wirbelknochen und in dem Fixierungskörper zu bilden.

2. Vorrichtung nach Anspruch 1,
**dadurch gekennzeichnet,**
**dass** das flexible Band (4, 4') einen Ankerstich (N) zur Fixierung am Wirbelknochen umfasst, wobei die zwei Knotenstränge (B) auf ein und derselben Seite des Stiftes (2) in dem Fixierungskörper (3) durch die Durchlaufschlitze (17) geführt werden.

3. Vorrichtung nach einem der vorhergehenden Ansprüche,
**dadurch gekennzeichnet,**
**dass** die Lamelle (6') in der Schlaufe (5') enthalten ist.

4. Vorrichtung nach einem der Ansprüche 1 und 2,
**dadurch gekennzeichnet,**
**dass** die Lamelle (6) außerhalb der Schlaufe (5) befestigt ist.

5. Vorrichtung nach einem der vorhergehenden Ansprüche,
**dadurch gekennzeichnet,**
**dass** die Mittel (M) zur einstellbaren Arretierung durch ein Verbindungsstück (21) gebildet sind, das die gegenüberliegenden Enden der zwei U-Schenkel verbindet, dass die Schlitze (17) des Fixierungskörpers für die Durchführung des Bandes durch jeweilige gegenüberliegende, auf der Seite des U-Bodens ausgebildete Ausnehmungen gebildet sind, wobei die Arretierungsmittel (M) angeordnet sind, um den Stift gegen die Bodenwand des U zu pressen, und die gegenüberliegenden Ausnehmungen auf der Seite des U-Bodens für die Durchführung zwischen dem Boden und dem Stift angeordnet sind.

6. Vorrichtung nach Anspruch 5,
**dadurch gekennzeichnet,**
**dass** der Körper (3) einstückig ausgebildet ist.

7. Vorrichtung nach Anspruch 6,
**dadurch gekennzeichnet,**
**dass** der U-Boden eine Bodenwand (14) bildet, dass diese Wand (10) die Form eines komplementär zu der Form des Stiftes ausgebildeten Halbzylinders aufweist, der beidseitig mit längsseitigen Rändern (16) endet, welche durch Verformung der Schenkel ein Einclipsen des Stifts (2) in den U-Boden zulassen.

8. Vorrichtung nach einem der Ansprüche 5 bis 7,
**dadurch gekennzeichnet,**
**dass** das Verbindungsstück (21) durch eine Klemmschraube gebildet ist, die auf der einen Seite mit einem durch einen ersten Schenkel des U durchgeführten Kopf versehen ist, wobei der Kopf einen kegelstumpfförmigen Abschnitt (24) aufweist, der angeordnet ist, um den Stift bei fortschreitender Verschraubung zusammenzudrücken, und auf der anderen Seite mit einem Ende (23) für die Verschraubung an dem entgegengesetzten U-Schenkel versehen ist.

9. Vorrichtung nach Anspruch 8,
**dadurch gekennzeichnet,**
**dass** der Körper (3) mit einem U-förmigen Querschnitt einen ersten U-Schenkel mit einer Außenfläche aufweist und dass der Kopf (22) der Klemmschraube ferner einen Absatz (26) aufweist, der geeignet ist, mit der Außenfläche (27) des ersten U-Schenkels zusammenzuwirken.

10. Vorrichtung nach einem der vorhergehenden Ansprüche,
**dadurch gekennzeichnet,**
**dass** der Fixierungskörper (3) aus einem Polymermaterial besteht, wobei der Stift (2) und das Verbindungsstück (21) aus Titan bestehen und es sich bei dem flexiblen Band (4) um ein Polymergeflecht handelt.

## Claims

1. Vertebral fixing device (1) for holding a spinal vertebra (V) on a rod (2), having said rod, a fixing body (3) of U-shaped or substantially U-shaped transverse section, a flexible band (4, 4') for connecting said vertebra with the fixing body, said fixing body having two slots (17) for passage of said band and means (M) for adjustable locking of the flexible band against the rod in said fixing body, **characterised in that** the flexible band (4, 4') forms a loop (5, 5') which is closed on itself and provided with a deformable strip (6, 6') made of flexible metal retaining its shape through flexion, such that it is capable of forming a guide for passage around the vertebra and into the fixing body.

2. Device according to claim 1, **characterised in that** the flexible band (4, 4') comprises a crown knot (N), for fixing to the vertebra, the two ends (B) emerging from the knot passing on the same side of the rod (2) into the fixing body (3) via the slots (17) for passage thereof.

3. Device according to any one of the preceding claims, **characterised in that** the strip (6') is included in the loop (5').

4. Device according to any one of claims 1 and 2, **characterised in that** the strip (6) is fixed to the exterior of the loop (5).

5. Device according to any one of the preceding claims, **characterised in that** the means (M) for adjustable locking are formed by a connecting part (21) linking the facing ends of the two branches of the U,
**in that** the slots (17) of the fixing body, for passage of said band, are each formed respectively by a facing recess situated on the side of the bottom of the U, the locking means (M) being arranged to compress the rod against the wall of the bottom of the U and the facing recesses being situated on the side of the bottom of the U for the passage between the said bottom and the rod.

6. Device according to claim 5, **characterised in that** the body (3) is formed in one piece.

7. Device according to claim 6, **characterised in that** the bottom of the U forming a bottom wall (14), said wall is in the shape of a half cylinder with a shape complementing that of the rod, terminated on either side by longitudinal edges (16) allowing the rod (2) to be clipped into the bottom of the U thanks to a deformation of the branches.

8. Device according to any one of claims 5 to 7, **characterised in that** the connecting part (21) is formed by a clamping screw provided on one side with a head (22) for passage through a first branch of the U, said head having a part with the shape of a truncated cone (24) arranged to compress the rod as it is screwed in, and on the other side with an end (23) for screwing to the opposite branch of the U.

9. Device according to claim 8, **characterised in that** the body (3) of U-shaped transverse section forms a first branch of the U comprising an external face and **in that** the head (22) of the clamping screw additionally has a shoulder (26) designed to co-operate with the external face (27) of the first branch of the U.

10. Device according to any one of the preceding claims, **characterised in that** the fixing body (3) is made of a polymer material, the rod (2) and the connecting part (21) being made of titanium and the flexible band (4) being a braid made of polymer.
